# EUROPÄISCHE PATENTSCHRIFT

(11) **EP 0 504 189 B1**
(45) Veröffentlichungstag und Bekanntmachung des Hinweises auf die Patenterteilung: **26.01.1994**
(21) Anmeldenummer: 91900009.1
(22) Anmeldetag: 07.12.1990
(51) Int. Cl.: A61F 2/36

(54) **ENDOPROTHESE**
ENDOPROSTHESIS
ENDOPROTHESE

(30) Priorität: 07.12.1989 DE 3940774
(43) Veröffentlichungstag der Anmeldung: 23.09.1992
(73) Patentinhaber: ZAHEDI, Amir, D-48143 Münster (DE)
(72) Erfinder: ZAHEDI, Amir, D-48143 Münster (DE)
(74) Vertreter: Habbel, Hans-Georg, Dipl.-Ing.
(86) Internationale Anmeldenummer: DE9000952
(87) Internationale Veröffentlichungsnummer: WO9108721

(56) Entgegenhaltungen:
- EP-A- 0 229 578
- EP-A- 0 311 208
- FR-A- 2 610 823
- US-A- 4 778 474

## Beschreibung

Die Erfindung betrifft eine Endoprothese der im Oberbegriff des Anspruches 1 angegebenen Art.

Derartige Endoprothesen sind beispielsweise aus der EP-0 229 578 Al bekannt. Sie weisen einen Schaft auf, der über seine Länge eingeschnitten ist, so daß er aus wenigstens zwei stiftartigen Bereichen besteht. Im verformten Zustand liegen die einzelnen Stifte nahe aneinander. Nach Einsetzen in den Knochen spreizen sich unter Temperatureinwirkung die stiftartigen Bereiche etwa ähnlich einem Spreizdübel , so daß insbesondere am distalen Ende der Endoprothese der Klemmdruck für die Verankerung aufgebaut wird.

Gerade am distalen Bereich liegt eine derartige Endoprothese, wenn sie beispielsweise als Femurendoprothese dient, dem Knochen im Bereich seiner geringsten Wandstärken an. Hier kann der Knochen demzufolge keine großen Kräfte aufnehmen, so daß hier die Gefahr von Beschädigungen des Knochengewebes besteht.

Weiterhin ist bei zementfreien Prothesen eine möglichst großflächige Anlage der Prothese am Knochen erwünscht, da der Knochenzement als Vermittler für die Kraftübertragung von der Prothese auf den Knochen fehlt und durch eine großflächige Anlage der Prothese am Knochen Überlastungen des Knochens verhindert werden sollen.

Der Erfindung liegt die Aufgabe zugrunde, bei einer Endoprothese der eingangs genannten Gattung die Verankerungssicherheit sowie die Formanpassung an gegebene anatomische Verhältnisse und an individuelle Größenunterschiede und Besonderheiten zu verbessern.

Diese Aufgabe wird mit den kennzeichnenden Merkmalen des Anspruches 1 gelöst.

Durch das Vorsehen von nach der Implantation expandierenden Bereichen im Schaftbereich ist die Ausräumung von weniger Knochenmaterial als bisher erforderlich. Die Wandstärke des Knochens ist in diesem Schaftbereich erheblich größer als am distalen Ende des Schaftes, so daß eine bessere Verankerung der Prothese erzielt wird. Insbesondere sind dabei durch Wärmerückstellung expandierende Bereiche im Bereich von Krümmungen vorgesehen. Auf diese Weise ist eine Anpassung an die unterschiedlichsten anatomischen Gegebenheiten auf einfache Weise möglich. Dabei sind die expandierenden Bereiche an der Innenseite von Krümmungsbereichen am Anfang und Ende der gekrümmten Zone und entsprechend an der Außenseite von Krümmungen in deren mittleren Zonen vorzusehen. Auf diese Weise weist dann das gesamte gekrümmte Element in eingefrorenem Zustand eine geringere Krümmung auf als in rückgestelltem, so daß einerseits beim Einbringen wenig Knochenmaterial entfernt werden muß und trotzdem andererseits ein fester hohlraumausfüllender Sitz des Implantats gewährleistet ist. Entsprechendes gilt, wenn der wärmerückstellfähige Bereich dort vorgesehen ist, wo sich bei eingesetzter Prothese eine Knochenausnehmung im Markraumbereich befindet, wie sie beispielsweise durch den großen oder kleinen Trochanter gebildet wird. Durch die Wärmerückstellung fügt sich die prothese in den betreffenden Bereich zusätzlich formschlüssig ein, was ebenfalls zur Verbesserung ihres Sitzes beiträgt.

Besonders vorteilhaft ist der erfindungsgemäße Schaft "mit Formgedächtnis" auch wegen der verringerten Genauigkeitsanforderungen an die Ausraspelung der Knochenhöhlung, da der Schaft sich während des Erwärmungsvorrganges weitgehend an die Höhlung anpaßt und innerhalb dieser verklemmt.

Entsprechend der Erfindung bildet das wärmerückstellfähige Material Teil eines vom Schaft abtrennbaren Elements. Diese abtrennbaren Elemente und der Schaft weisen Verbindungsvorrichtungen auf, mittels denen die abtrennbaren Elemente mit dem Schaft zusammenfügbar sind. Der Schaft ist mit einer Bohrung versehen, in die eine mit dem abtrennbaren Element verbundene Nase oder ein mit dem abtrennbaren Element verbundener Stift eingreift. zudem weist der Schaft in die Außenkontur eingelassene Ausnehmungen auf, in der die abtrennbaren Elemente aufgenommen werden. Diese abtrennbaren Elemente sind spangenförmig ausgebildet und sind mindestens teilweise aus wärmerückstellfähigem Material. Die spangenförmigen Elemente sind derart ausgebildet, daß sie im fixierten Zustand in den Schaft drehsicher einsteckbar sind. Im rückgestellten Zustand weisen die spangenförmigen Elemente mindestens einen Teilbereich mit einer konvexen Wölbung auf, wodurch der effektive Querschnitt der Endoprothese vergrößert und eine bessere Anpassung an den Knocheninnenraum ermöglicht wird. Durch diese Ausführungsform der abtrennbaren Elemente ist die Einführung und Anpassung einer solchen Endoprothese in eine längsgekrümmte Knochenhöhlung erheblich leichter und besser durchführbar als bei den bekannten Endoprothesen.

Bei einer vorteilhaften Weiterbildung der Erfindung ist das abtrennbare Element in Form von mehreren eine gemeinsame verbindungsvorrichtung aufweisenden parallelen Teilen ausgebildet. Diese parallelen Teile sind an beiden Enden in Aufnahmeköpfe gefaßt, wobei der proximale Aufnahmekopf des abtrennbaren Elements als Teil einer verbindungsvorrichtung ausgebildet ist, welche in die durchgehende Bohrung des Schafts verdrehungssicher einsteckbar ist und der distale Aufnahmekopf am Schaft gleitend anliegt.

Durch die größen- und ortsvariabel patientenindividuell auswähl- und einsetzbaren Zusatzelemente kann eine herkömmliche Serienprothese zu einer "quasiindividuellen" Prothese verfeinert werden, so daß sie in ihren Eigenschaften und insbesondere hinsichtlich der Festigkeit des Knochenanschlusses mit mittels computergesteuerten Werkzeugmaschinen individuell aus massivem Material hergestellten und an den Knocheninnenraum angepaßten Prothesenschäften vergleichbar ist. Bei anderen bevorzugten Ausführungen sind die wärmerückstellfähigen Bereiche insbesondere dorsal und/oder ventral vorgesehen, da sich in diesen Bereichen eine optimale Verklemmung erzielen läßt.

Als Materialien, denen ein Formgedächtnis eingeprägt werden kann, eignen sich prinzipiell sämtliche amorphe und teilkristalline Kunststoffe sowie Metall-Legierungen, wobei zur Herstellung von Implantaten nur körperverträgliche Stoffe in Frage kommen. Demzufolge sind bei Prothesenschäften mit rückstellfähigem Material aufweisender Oberfläche vorzugsweise Polyethylen, Teflon, Polyacetat, Silicon oder Titan-Nickel-Cobalt-Legierung als rückstellfähiges Material bildende Substanzen einzusetzen. Die für das Rückstellverhalten erforderliche Fixierung einer Molekül- bzw. Kristallverkettung oder -vernetzung, die das Formgedächtnis erzeugt, erfolgt je nach verwendetem Material bei einer Fixiertemperatur, die der gewünschten Rückstelltemperatur entspricht, unter Ausübung eines hohen Druckes. Nach der Abkühlung bewirkte Formänderungen, beispielsweise durch verbiegen, führen zu einer Dehnung bzw. Stauchung, nicht aber zu einem Aufbrechen der Verkettungs-oder Vernetzungsstrukturen, so daß bei Erwärmung auf die Fixier bzw. Rückstelltemperatur Rückstellkräfte aktiviert werden, durch die der Prothesenschaft seine ursprüngliche Form wieder einnimmt. Die Fixier- bzw. Rückstelltemperatur kann bei Prothesenmaterialien mit entsprechenden molekularen bzw. kristallinen Eigenschaften sehr niedrig, insbesondere bei etwa 50°C, gewählt werden, so daß der kurzzeitige und lokal eng begrenzte Erwärmungsprozeß keine Gewebeschädigung verursacht. Es ist aber auch möglich, eine der Körpertemperatur entsprechende Fixier- bzw. Rückstelltemperatur zu wählen und die Formanpassung des abtrennbaren Elements bei einer niedrigeren Temperatur durchzuführen, so daß eine zusätzliche Wärmezufuhr zum Auslösen des Rückstellprozesses des eingesetzten Prothesenschaftes nicht erforderlich ist.

Das wärmerückstellfähige Material kann auch Teil eines Verbundwerkstoffes sein und vorzugsweise in Form im wesentlichen parallel angeordneten und sich in Längsrichtung der abtrennbaren Elemente erstreckenden faserartigen Elementen oder sich überkreuzenden faserartigen Element en ausgebild et sein. Derartige faserartige Elemente sind bevorzugt in einer oberflächennahen Schicht der abtrennbaren Elemente eingearbeitet, so daß sie allseitig von Elementmaterial umschlossen sind. In diesem Fall kann das verwendete wärmerückstellfähige Material auch aus körperunverträglicher Metall-Legierung oder körperunverträglichem Kunststoff, insbesondere Elastomer, insbesondere Polychloropren oder Ethylen-Propylen-Kautschuk, bestehen. Durch verbiegen und schmiedeartigem Bearbeiten bei unterhalb der Fixations- und Rückstelltemperatur liegender Verformungstemperatur läßt sich das abtrennbare Element derart begradigen und von wulstartigen Verdickungen befreien, daß ein leichtes Einsetzen der Prothese in eine entsprechend vorbereitete Knochenhöhlung möglich ist. Die wärmerückstellfähigen faserartigen Elemente des abtrennbaren Elements bewirkt, daß sich durch Wärmezufuhr - vorzugsweise Körperwärme - anschließend der gesamte Schaft in die ursprüngliche Form zurückbildet. vorteilhafte Weiterbildungen der Erfindung sind in den Unteransprüchen gekennzeichnet bzw. werden nachstehend zusammen mit der Beschreibung der bevorzugten Ausführung der Erfindung anhand mehrerer Schnittfiguren näher dargestellt. Es zeigen:
Figur 1 einen zur Implantation einer Femurkomponente einer Alloarthroplastik vorbereiteten Femurknochen,
Figur 2 eine zur Implantation in den Femurknochen gemäß Figur 1 ausgeführte Femurkomponente,
Figur 9 eine Seitenansicht einer zur Implantation in den Femurknochen gemäß Figur 1 erfindungsgemäßen Ausführungsform einer Femurkomponente im verformten Zustand,
Figur 10 einen Teillängsschnitt durch die Femurkomponente gemäß Figur 9 linksseitig im wärmerückverformten und rechtsseitig im verformten Zustand,
Figuren 13 und 14 eine Ansicht eines abtrennbaren im verformten und wärmerückverformten Zustand,
Figur 15 eine erste Ausführungsform eines verbundwerkstoffs und
Figur 16 eine zweite Ausführungsform eines verbundwerkstoffs.

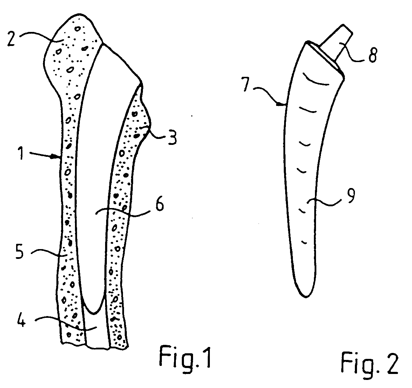

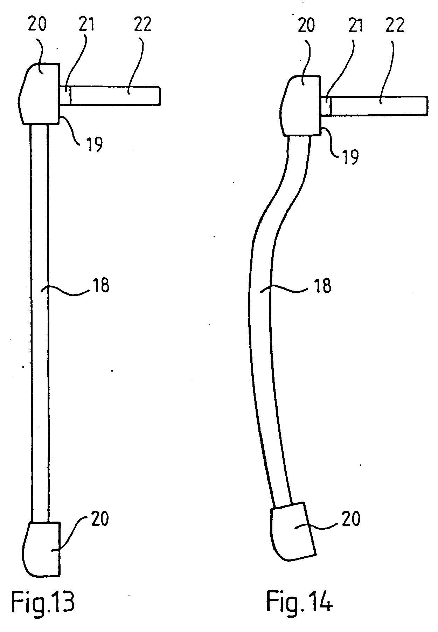

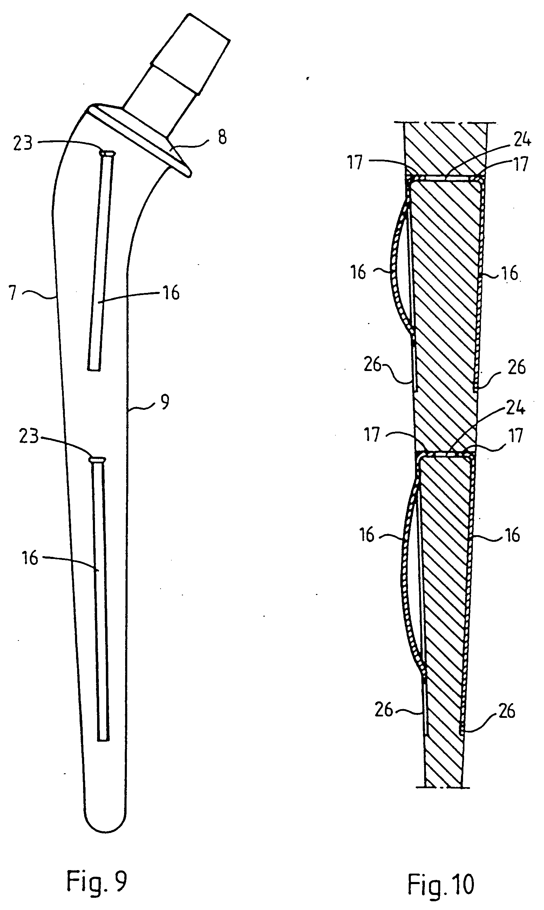

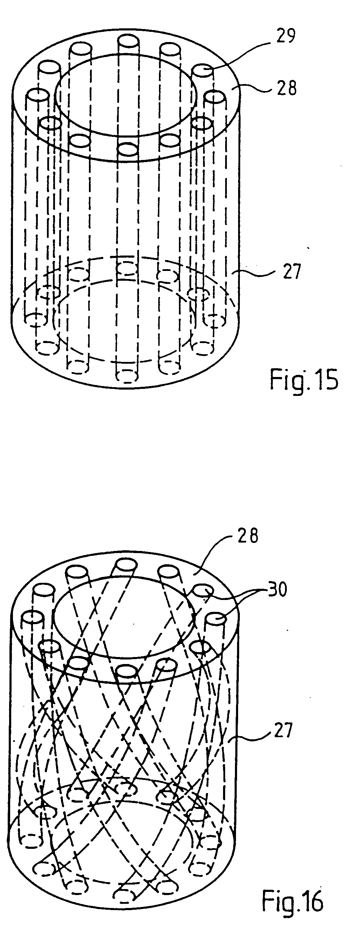

In Figur 1 ist ein Längsschnitt durch einen Femurknochen 1 mit angedeutetem Trochanter major 2 sowie Trochanter minor 3 wiedergegeben. Ein Teil des Markraumes 4 und ein geringer Teil der den Markraum 4 umgebenden Kortikalis 5 sind in Form einer Aushöhlung 6 ausgeraspelt, wobei Größe und Form der Aushöhlung 6 etwa der eines Schaftes einer in dieser Höhlung 6 einzusetzenden Femurkomponente einer Alloarthroplastik angepaßt sind.

Eine derartige Femurkomponente 7 mit einem halsartigen Vorsprung 8 zur Aufnahme einer - nicht dargestellten - Gelenkkugel und einem Schaft 9 ist in Figur 2 dargestellt.

Eine Ausführungsform einer Femurkomponente 7 mit einem halsartigen Vorsprung 8 zur Aufnahme einer Gelenkkugel und einem Schaft 9 entsprechend der Erfindung ist in den Figuren 9 und 10 dargestellt. Der Schaft 9 der Femurkomponente 7 ist spiegelsymmetrisch ausgebildet und kann sowohl für die linke als auch für die rechte Körperhälfte eingesetzt werden und besteht aus biokompatiblem Material. Der Schaft 9 weist zwei als durchgehende Bohrungen 24 ausgebildete Aussparungen 23 und in der Schaftoberfläche eingelassene Ausnehmungen 26 auf. Auf zwei gegenüberliegenden Seiten des Schafts 9 sind abtrennbare Elemente 16 jeweils mittels einer als Nase 17 ausgebildeten und mit der mit dem abtrennbaren Element 16 verbundenen Verbindungsvorrichtung 19, die in die durchgehende Bohrung 24 des Schafts 9 eingreift, lösbar zusammengefügt. Die abtrennbaren Elemente 16 aus biokompatiblem und wärmerückstellfähigem Material, weisen außer der als Teil der Verbindungsvorrichtung diendenden Nase 17 eine gradlinig verlaufende stabartige Form auf, die durch äußere mechanische Einwirkungen nach Art einer Streckung, ausgehend von der ursprünglich konvex gewölbten Form, erzeugt worden ist. Die Nase 17 des abtrennbaren Elements 16 ist im wesentlichen quer zum gradlinig verlaufenden Teil des abtrennbaren Elements 16 ausgerichtet und weist eine nicht kreisrunde Querschnittsform auf. Im fixiert verformten Zustand vor Einbringung der Endoprothese in den Knochenin- nenraum befindet sich die als Teil der verbindungsvorrichtung 19 dienende Nase 17 des jeweiligen abtrennbaren Elements 16 drehsicher in den entsprechenden als durchgehende Bohrung 24 ausgebildeten Teil der verbindungsvorrichtung 19 des Schafts 9 und der gradlinig verlaufende Teil des abtrennbaren Elements 16 liegt gleitend in einer in der Schaftoberfläche eingelassenen Ausnehmung 26 an. Die Oberfläche der Endoprothese weist eine kontinuierliche Gestaltung auf.

In Figur 10 ist der ursprüngliche Ausgangszustand der abtrennbaren Elemente 16 linksseitig dargestellt, welche sie nach der Einbringung, durch die Aktivierung der Rückstellkräfte mittels einer Zufuhr von Wärmeenergie beim Erreichen der Fixier- und Rückstelltemperatur wieder annehmen. Bei dieser Temperatur bilden die abtrennbaren und spangenförmig ausgebildeten Elemente 16, aufgrund deren durch Stabilisierung der Gitter-bzw. Molekülstruktur erzeugten Formgedächtnis-Eigenschaften, ihre Form in den Ausgangszustand zurück. Um gewebeschädigende, starke Erwärmung zu vermeiden, ist die Fixier- und Rückstelltemperatur zweckmäßigerweise in einem Bereich vorgewählt, der die Körpertemperatur nicht oder nur geringfügig überschreitet. Im rückgestellten, linksseitig dargestellten Zustand weist das proximale spangenförmige abtrennbare Element 16 eine konvexe Wölbung mit über ihre freie Länge zum Befestigungspunkt abnehmender Krümmung auf, wogegen das distale abtrennbare Element 16 eine konvexe Wölbung mit einer im wesentlichen konstanten Krümmung aufweist. Durch diese Art der Wölbungen nehmen die spangenförmigen Elemente 16 im rückgestellten Zustand eine Form an, welche in günstiger Weise mit der natürlichen Form des Knocheninnenraums übereinstimmt.

Bei einer hier nicht dargestellten Weiterbildung dieser Ausführungsform ist die Ausnehmung 26 der Schaftoberfläche derart verkürzt ausgebildet, daß das spangenförmige Element 16 im fixiert verformten Zustand von der Ausnehmung 26 bündig aufgenommen wird. Beim Rückstellvorgang, bedingt durch die Dehnung des spangenförmigen Elements 16 werden dann zusätzliche Rückstellkräfte erzeugt und die Rückverformung dadurch gefördert.

Die Figuren 13 und 14 zeigen eine weitere Ausführungsform von spangenförmigen Elementen 16 mit Wärmerückstelleigenschaften, die jeweils in Form von mehreren eine gemeinsame Verbindungsvorrichtung 19 aufweisenden parallelen Teilen 18 ausgebildet sind. Diese parallelen Teile 18 sind aus biokompatiblem und wärmerückstellfähigem Material und weisen an den Enden gemeinsame Aufnahmeköpfe 20 auf, die einen Teil der Verbindungsvorrichtung 19 bilden. Der an der Außenseite verrundete Aufnahmekopf 20, der in einer zur Schaftoberfläche parallelen Ebene eine Paßscheibe 21 aufweist, die einen von der Kreisform abweichenden Querschnitt aufweist, trägt zudem einen Stift 22. Der Schaft 9 weist eine durchgehende Bohrung 24 auf, die einen Querschnitt aufweist, die an die Form des Stifts 22 und der Paßscheibe 21 angepaßt ist. Somit bildet die Paßscheibe 21 beim Eingreifen in die formangepaßte Ausnehmung 25 der durchgehenden Bohrung 24 eine Rotationssicherung. Die parallelen Teile 18 des spangenförmigen Elements 16 werden am freien Ende des spangenförmigen Elements 16 in einem gemeinsamen Aufnahmekopf 20 gehalten, der gleitend an der Oberfläche des Schafts 9 anliegt. In Figur 13 ist der fixierte verformte Zustand des spangenförmigen Elements 16 vor Einbringung in die Knochenpartie und in Figur 14 ist der rückgestellte Zustand des spangenförmigen Elements 16 nach Einbringung in die Knochenpartie dargestellt. Dabei weisen die parallelen Teile 18 des spangenförmigen Elements 16 eine konvexe Wölbung auf, die über ihre freie Länge zum Befestigungspunkt hin eine zunehmende Krümmung aufweist.

Zwei Varianten für die Zusammensetzung des verwendeten Verbundwerkstoffs für die Umhüllung 27 der abtrennbaren Elemente 16 sind in den Figuren 15 und 16 dargestellt. Gemäß Figur 15 besteht die Umhüllung 27 für das abtrennbare Element 16 aus einem Grundmaterial 28, vorteilhafterweise einem bewährten körperverträglichen Prothesenwerkstoff, und einer Anzahl im wesentlichen parallel angeordneter und sich in Längsrichtung des abtrennbaren Elements 16 erstreckender faserartiger schlanker Elemente 29, welche von dem Grundmaterial 28 allseitig eingeschlossen sind. Die faserartigen schlanken Elemente 29 bilden die Elemente mit Formgedächtnis, so daß durch ihre im wesentlichen gleichmäßige Verteilung innerhalb der Umhüllung 27 des abtrennbaren Elements 16 eine Rückstellkraft auch auf das Grundmaterial 28 des abtrennbaren Elements 16 ausgeübt wird. Dieser Mitmahmeeffekt wirkt bei der Parallelanordnung der faserartigen schlanken Elemente 29 vor allem in Längsrichtung des abtrennbaren Elements 16, d.h. daß sich eine reine Streckung oder auch eine Stauchung des abtrennbaren Elements 16 wieder rückgängig machen läßt. Das gilt für Biegungen oder geschmiedete Formveränderungen in weitaus geringerem Maße, da die radiale Komponente der Rückstellkraft bei derart separaten, nicht miteinander verbundenen, faserartigen schlanken Elementen 29 wesentlich geringer als die axiale Komponente ist.

Für Anwendungsfälle, bei denen eine Rückstellung in eine Vorzugsrichtung nicht ausreicht, ist das wärmerückstellfähige Material - wie Figur 16 zeigt - in Form sich überkreuzender faserartiger schlanker Elemente 30 in ein Grundmaterial 28 eingebettet. Auf diese Weise läßt sich ein in radialer und axialer Richtung wirkender Rückstelleffekt erzielen.

Die faserartigen schlanken Elemente 29 und 30 können, da sie in das Grundmaterial 28 der Umhüllung 27 eingeschlossen sind, auch aus nicht biokompatiblem Material bestehen. Demzufolge steht eine große Anzahl sehr verschiedener Materialien, die sich mit einem Formgedächtnis ausstatten lassen, zur Verfügung. Auswahlkriterien sind dann vor allem die Fixations- und Rückstelltemperatur sowie die Art der Verformung und der damit zusammenhängenden materialspezifischen Rückstellkräfte.

Es ist ersichtlich, daß die wärmerückstellfähigen Elemente oder Zonen bei anderen - nicht in der Zeichnung näher dargestellten Ausführungformen auch in Innenkrümmungen im Bereich der Enden der gekrümmten Zone und bei Außenkrümmungen im mittleren Bereich der gekrümmten Zone vorgesehen sein kann, so daß die jeweiligen Krümmungen erst im rückgestellten Zustand entsprechend den anantomischen Formen verlaufen und dabei insgesamt eine größere Krümmung aufweisen als in "eingefrorenem" Zustand der wärmerückstellbaren Zonen zu Einsetzen der Prothese, wobei durch diese Bereiche in Richtung Einführen der Prothese auch hohle Knocheninnenräume mit größerer Mindestkrümmung passiert werden können.

Entsprechend sind wärmerückstellbare Bereiche bevorzugt auch dort vorgesehen, wo die in den Knochen eingesetzte Prothese einer Knochenausnehmung benachbart ist. Durch Wärmerücksstellung schmiegt sich dieser Oberflächenbereich der Prothese dann besonders gut der Kontur der Knochenoberfläche an. Auf diese Weise kann die Prothesenoberfläche insgesamt nahezu beliebigen Oberflächenformen des Knochens - auch S-förmig gekrümmten - folgen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Endoprothese mit einem Schaft zur Verankerung in einem Röhrenknochen, welcher Schaft mindestens teilweise aus wärmerückstellfähigem, d. h. unter Wärmeeinwirkung in seine Ausgangsform wieder rückführbarem Material besteht, dadurch gekennzeichnet, daß der Schaft (9) eine in seine Außenkontur eingelassene Aussparung (23) und eine Ausnehmung (26) aufweist und daß das wärmerückstellfähige Material Teil eines abtrennbaren, spangenförmigen Elementes (16) bildet, wobei das spangenförmige Element (16) an seinem einen Ende eine in die Aussparung (23) einsteckbare Nase (17) oder einen in die Aussparung einsteckbaren Stift (22) aufweist und im gesteckten Zustand an seinem anderen Ende längsverschiebbar in der Ausnehmung (26) geführt ist.

2. Endoprothese nach Anspruch 1, dadurch gekennzeichnet, daß das wärmerückstellfähige Material in einem Bereich mit konvexer Wölbung (10) vorgesehen ist.

3. Endoprothese nach Anspruch 1 oder 2, dadurch gekennzeichnet, daß die Aussparung (23) eine durchgehende Bohrung (24) bildet.

4. Endoprothese nach einem der vorhergehenden Ansprüche, dadurch gekennzeichnet, daß das abtrennbare spangenförmige Element (16) insbesondere in Form von mehreren eine gemeinsame Verbindungsvorrichtung (19) aufweisenden parallelen Teilen (18) ausgebildet ist.

5. Endoprothese nach Anspruch 4, dadurch gekennzeichnet, daß das freie Ende des spangenförmigen Elements (16) an der Oberfläche des Schafts (9) anliegt oder von einer entsprechenden Ausnehmung (26) der Oberfläche bündig aufgenommen wird.

6. Endoprothese nach einem der vorhergehenden Ansprüche, dadurch gekennzeichnet, daß das abtrennbare Element einen die Nase (17) oder den Stift (22) tragenden, insbesondere an der Außenseite verrundeten, einen Teil der Verbindungsvorrichtung (19) bildenden, Aufnahmekopf (20) aufweist, der in einer zur Schaftoberfläche parallelen Ebene einen von der Kreisform abweichenden Querschnitt aufweist und der, insbesondere in Form einer Paßscheibe (21), in eine formangepaßte Ausnehmung (25) der Schaftoberfläche eingreift und damit eine Rotationssicherung bildet.

7. Endoprothese nach Anspruch 4, dadurch gekennzeichnet, daß das spangenförmige Element (16) im rückgestellten Zustand eine konvexe Wölbung mit über ihre freie Länge im wesentlichen konstanter Krümmung oder mit zum Befestigungspunkt zunehmender Krümmung aufweisen.

8. Endoprothese nach einem der vorhergehenden Ansprüche, dadurch gekennzeichnet, daß das wärmerückstellfähige Material aus mindestens einer biokompatiblen Metall-Legierung, insbesondere Titan-Nickel-Cobalt-Legierung, oder einem Kunststoff, insbesondere Elastomer, insbesondere Polychloropren oder Ethylen-Propylen-Kautschuk, besteht.

9. Endoprothese nach Anspruch 8, dadurch gekennzeichnet, daß das wärmerückstellfähige Material, insbesondere in Form von sich vorzugsweise parallel oder einander überkreuzend in Schaftlängsrichtung erstreckenden faserartigen oder in sonstiger Weise schlanken Elementen Teil eines Verbundwerkstoffes bildet.

10. Endoprothese nach einem der vorhergehenden Ansprüche, dadurch gekennzeichnet, daß die Matrix des Verbundwerkstoffs aus mindestens einem biokompatiblen Kunststoff, insbesondere Polyethylen, Teflon, Polyacetal oder Silicon, besteht.

11. Endoprothese nach einem der vorhergehenden Ansprüche, dadurch gekennzeichnet, daß die Fixier- und Rückstelltemperatur des wärmerückstellfähigen Materials nicht oder nur geringfügig oberhalb der Körpertemperatur liegt.

12. Endoprothese nach einem der vorhergehenden Ansprüche, dadurch gekennzeichnet, daß wärmerückstellfähige Bereiche in Innenkrümmungen im Bereich der Enden der gekrümmten Zone und bei Außenkrümmungen im mittleren Bereich der gekrümmten Zone oder in einem Bereich vorgesehen ist, der bei in den Knochen eingesetzter Prothese einer Knochenausnehmung benachbart ist.

## Claims

1. An endoprosthesis comprising a shaft for anchoring in a tubular bone, which shaft consists at least partially of heat-restorable material, i.e. material which can be returned to its initial form under the action of heat, characterized in that the shaft (9) comprises a recess (23) sunk into its outer contour and a groove (26) and in that the heat-restorable material forms part of a separable, clasp-like member (16), the clasp-like member (16) comprising, at one of its ends, a lug (17) insertable into the recess (23) or a pin (22) insertable into the recess and being guided longitudinally displaceably in the groove (26) at its other end when in the inserted state.

2. An endoprosthesis according to claim 1, characterized in that the heat-restorable material is provided in one area with a convex bulge (10).

3. An endoprosthesis according to claim 1 or claim 2, characterized in that the recess (23) forms a through-hole (24).

4. An endoprosthesis according to any one of the preceding claims, characterized in that the separable, clasp-like member (16) is constructed in particular in the form of a plurality of parallel parts (18) having a common connecting device (19).

5. An endoprosthesis according to claim 4, characterized in that the free end of the clasp-like member (16) rests on the surface of the shaft (9) or is accommodated in flush manner by a corresponding groove (26) in the surface.

6. An endoprosthesis according to any one of the preceding claims, characterized in that the separable member comprises a receiving head (20) carrying the lug (17) or the pin (22), rounded, especially on the outside, and forming part of the connecting device (19), which receiving head (20) has a cross-section deviating from the circular in a plane parallel with the shaft surface and which, especially in the form of a shim ring (21), engages in a shape-conformed groove (25) in the shaft surface and thus forms a rotation safety device.

7. An endoprosthesis according to claim 4, characterized in that, in the restored state, the clasp-like member (16) comprises a convex bulge with a curvature which is substantially constant over its free length or with a curvature increasing towards the fastening point.

8. An endoprosthesis according to any one of the preceding claims, characterized in that the heat-restorable material consists of at least one bio-compatible metal alloy, especially titanium/nickel/cobalt alloy, or a plastics maaterial, especially an elastomer, especially polychloroprene or ethylene/propylene rubber.

9. An endoprosthesis according to claim 8, characterized in that the heat-restorable material, especially in the form of fibre-type or otherwise slim members extending in the longitudinal direction of the shaft, preferably in parallel or mutually crossed, forms part of a composite material.

10. An endoprosthesis according to any one of the preceding claims, characterized in that the matrix of the composite material consists of at least one bio-compatible plastics material, especially polyethylene, teflon, polyacetal or silicone.

11. An endoprosthesis according to any one of the preceding claims, characterized in that the fixing and restoring temperature of the heat-restorable material does not lie or lies only slightly above body temperature.

12. An endoprosthesis according to any one of the preceding claims, characterized in that heat-restorable areas are provided in internal curves in the area of the ends of the curved zone and for external curves in the central area of the curved zone or in an area which, when the prosthesis is inserted into the bone, is adjacent a bone recess.

## Revendications

1. Endoprothèse comportant une tige pour l'ancrage dans un os tubulaire, laquelle tige est composée, au moins en partie, d'une matière susceptible de reprise de forme thermique, c'est-à-dire qui peut être ramenée à sa forme d'origine par l'action de la chaleur, caractérisée en ce que la tige (9) présente une cavité (23) ménagée dans son contour extérieur et un évidement (26), et en ce que la matière susceptible de reprise de forme thermique forme une partie d'un élément amovible (16) en forme de boucle, l'élément (16) en forme de boucle présentant, à l'une de ses extrémités, une dent (17) qui peut être emmanchée dans la cavité (23) ou une cheville (22) pouvant être enfoncée dans la cavité, et étant guidé au niveau de son extrémité, mobile en coulissement longitudinal dans l'évidement (26) dans l'état mis en place.

2. Endoprothèse selon la revendication 1, caractérisée en ce que la matière susceptible de reprise de forme thermique est munie d'une région possédant un bombé convexe (10).

3. Endoprothèse selon la revendication 1 ou 2, caractérisée en ce que la cavité (23) forme un perçage traversant (24).

4. Endoprothèse selon l'une des revendications précédentes, caractérisée en ce que l'élément amovible (16) en forme de boucle est réalisé en particulier sous la forme de plusieurs parties (18) présentant un dispositif d'assemblage commun (19).

5. Endoprothèse selon la revendication 4, caractérisée en ce que l'extrémité libre de l'élément (16) en forme de boucle est appuyée contre la surface de la tige (9) ou est logée dans un évidement correspondant (26) de la surface de façon à se trouver à l'affleurement.

6. Endoprothèse selon l'une des revendications précédentes, caractérisée en ce que l'élément amovible présente une tête réceptrice (20) qui porte la dent (17) ou la cheville (22), en particulier, arrondie sur sa face extérieure, et qui forme une partie du dispositif d'assemblage (19), cette tête présentant, dans un plan parallèle à la surface de la tige, une section transversale différente de la forme circulaire, et qui est engagée, en particulier sous la forme d'une rondelle ajustée (21), dans un évidement (25) de la surface de la tige qui y est adapté par sa forme, et forme ainsi un blocage en rotation.

7. Endoprothèse selon la revendication 4, caractérisée en ce que l'élément (16) en forme de boucle présente, à l'état de reprise de forme, un bombé convexe possédant une courbure sensiblement constante sur toute sa longueur libre, ou une courbure qui croît en direction de son point de fixation.

8. Endoprothèse selon l'une des revendications précédentes, caractérisée en ce que la matière susceptible de reprise thermique est composée d'au moins un alliage métallique biocompatible, en particulier d'un alliage titane-nickel-cobalt, ou d'une matière plastique, notamment d'un élastomère, en particulier d'un polychloroprène ou d'un caoutchouc éthylène-propylène.

9. Endoprothèse selon la revendication 8, caractérisée en ce que la matière susceptible de reprise de forme thermique, notamment composée d'éléments fibreux ou, de toute façon minces, qui s'étendent de préférence parallèlement entre eux ou en se croisant mutuellement dans la direction longitudinale de la tige, forme une partie d'un matériau composite.

10. Endoprothèse selon l'une des revendications précédentes, caractérisée en ce que la matrice du matériau composite est formée d'au moins une matière plastique biocompatible, notamment de polyéthylène, Téflon, polyacétal ou silicone.

11. Endoprothèse selon l'une des revendications précédentes, caractérisée en ce que la température de fixation et de reprise de forme de la matière susceptible de reprise de forme thermique n'est pas supérieure à la température du corps ou n'est pas légèrement supérieure à cette température.

12. Endoprothèse selon l'une des revendications précédentes, caractérisée en ce que, dans des courbures intérieures, les régions susceptibles de reprise de forme thermique sont situées dans la région des extrémités de la zone courbée tandis que, dans des courbures extérieures, ces régions sont prévues dans la région centrale de la zone courbée ou dans une région qui est adjacente à un évidement de l'os lorsque la prothèse est mise en place dans l'os.
